# EUROPEAN PATENT APPLICATION

(11) **EP 3 900 698 A1**
(43) Date of publication of application: **27.10.2021**
(21) Application number: 20171117.3
(22) Date of filing: 23.04.2020
(51) Int. Cl.: A61K 8/19, A61K 8/26, A61K 8/86, A61K 8/97, A61Q 19/10

(54) **PRODUCT AND PROCESS FOR DECONTAMINATION OF HARMFUL SUBSTANCES FROM THE SKIN**

(71) Applicant: Leibniz-Institut für Polymerforschung Dresden e.V., 01069 Dresden (DE)
(72) Inventor: GUPTA, Vaibhav, 01067 Dresden (DE); SCHUBERT, Jonas, 01099 Dresden (DE); SCHNEPF, Max, 01099 Dresden (DE)
(74) Representative: Maikowski & Ninnemann Patentanwälte Partnerschaft mbB

(57) **Abstract**

The present invention relates to the field of skin decontamination from harmful substances. In particular, the invention provides composition for decontamination of skin from hazardous substances, such as nanoparticles, microplastic particles and viruses, wherein said composition comprises
• 1 to 40 % by weight of at least one water-soluble polymer,
• 1 to 40 % by weight of at least one phyllosilicate,
• 1 o 30 % by weight charcoal and/or graphite, and
• water.

In further embodiment, the invention provides a "ready to use" kit comprising said composition, which enables more than 99% decontamination of affected skin areas within the very important first minutes. The kit allows the use with one hand, thus significantly reducing the risk of secondary contamination of other skin areas. In further embodiment, the invention also relates to a process for skin decontamination from hazardous substances, such as nanoparticles, microplastic particles and viruses comprising the composition and/or kit of the invention.

## Description

### Field of the invention

The present invention relates to the field of skin decontamination from harmful substances. In particular, the invention provides composition for decontamination of skin from nanoparticles, wherein said composition comprises
- 1 to 40 % by weight of at least one water-soluble polymer,
- 1 to 40 % by weight of at least one phyllosilicate,
- 1 o 30 % by weight charcoal and/or graphite, and
- water.
The composition of the invention is particularly advantageous, because said composition does not affect the barrier function of the skin. In a preferred embodiment, the composition of the invention does not contain surfactants. In further embodiment, the invention provides a "ready to use" kit comprising said composition, which enables more than 99% decontamination of affected skin areas within the very important first minutes. The kit allows the use with one hand, thus significantly reducing the risk of secondary contamination of other skin areas. In further embodiment, the invention also relates to a method for skin decontamination from nanoparticles comprising the composition and/or kit of the invention.

### Background of the invention

For about 10 years hazardous compounds such as nanoparticles have been increasingly used in industry and science. Nanoparticles have made the leap into widespread use and are, depending on the compound class, used in many different fields, for example as semiconductors in electronics, in various materials, paints and varnishes. The high reactivity of nanoparticles and the increase in production and application of different types of nanoparticles represent a still insufficiently known risk. Also the occupational safety with regard to nanomaterials has not yet reached a uniform standard. It is known from a few studies on this topic (Lewinski, N., Berthet, A., Maurizi, L., Eisenbeis, A., & Hopf, N. (2017); Effectiveness of hand washing on the removal of iron oxide nanoparticles from human skin ex vivo. Journal Of Occupational And Environmental Hygiene, 14(8), D115-D119) that in case of direct contamination of the skin with metallic or carbon nanoparticles, simple hand washing is not sufficient to remove the nanoparticles from the skin surface. The use of surfactants in particular is disadvantageous, as they promote penetration into the hair roots and deeper skin layers. Due to their small size, nanoparticles can be absorbed into the body through the skin and spread throughout the entire organism via the bloodstream. During the manufacture of products containing nanoparticles, employees come into contact with these potentially harmful substances. Numerous studies are currently being conducted on the risks associated with nanoparticles in industrial production and elesewhere. The NanoCare project (see https://www.nanopartikel.info/projekte/abgeschlossene-projekte/nanocare) has provided the first binding work instructions for the uniform measurement of nanoparticles. However, these focus primarily on airborne nanoparticles and less on skin contamination.

Nanoparticles can be found in more and more products and things of everyday life. According to studies, in 2015, approximately 4.1 billion dollars sales were achieved with products containing nanoparticles. Global sales of such products are expected to grow by 20% annually to as much as $11.3 billion in 2020. Despite high safety standards in handling nanoparticles, the risk of unintentional skin contact also increases due to their increasing use. Therefore, a first aid kit for rapid decontamination of skin from nanoparticles is becoming increasingly important not only in scientific laboratories but also in industrial production. A glance at history shows that the initially rather careless handling of new materials and substances can later have serious health consequences for the people affected. The example of asbestos shows that with new technologies, safe handling is not always guaranteed, especially at the beginning of the market launch. In the event of accidents, effective "first aid products" are needed.

Conventional treatment measures aim at decontaminating a specific substance. The decontamination effect of such formulations, such as the product Dekontafix® for radiopharmacy, is therefore limited to specific substances. In the case of contamination with fat-soluble substances, the substance polyethylene glycol (PEG) is used for rinsing in the laboratory. However, no product specifically for the decontamination of nanoparticles is available on the market.

A similar situation exists for other hazardous compounds such as microplastic particles, viruses etc.

CN 107 998 065 A teaches a composition for use as a face mask for cleaning the skin. The composition does not contain activated carbon. Moreover, the use of this composition for the decontamination of nanoparticles in the field of occupational safety is not evident from CN 107 998 065 A.

The teaching of EP 2 470136 B1 can be considered in combination with the study of Lademann et al. (Lademann, J., Knarr, F., Patzelt, A., Meinke, M., Richter, H., Krutmann, J., Rühl, E. and Doucet, 0. (2018). Laser Scanning Microscopic Investigations of the Decontamination of Soot Nanoparticles from the Skin. Skin Pharmacology and Physiology, 31(2), pp.87-94) and has a similar purpose as the present invention. Nanoparticles are removed from the skin by an applicator containing absorbent carbon nanofibres. The study shows that the application of the cleanser is particularly effective if a protective cream is applied beforehand. This is disadvantageous, especially in the case of occupational accidents.

DE 10 2017 010 930 A1 teaches the use of carbon particles of a certain size for almost all conceivable cosmetic and medical purposes in topical preparations. Claim 1 includes "at least one type of carbon particles an average particle size of dso = 650 ± 200 nm". There is no explicit suitability for the removal of nanoparticles mentioned in the general disclosure and a composition designed for this purpose is also not disclosed. The compositions disclosed in DE 10 2017 010 930 A1 seem therefore not suitable for the decontamination of nanoparticles from skin.

### Description of the invention

The purpose of the present invention is therefore to provide a product and a process for rapid decontamination of skin surfaces from hazardous substances, such as nanoparticles, microplastic particles, viruses and the like. The product and process of the invention are especially suitable for the rapid decontamination of skin surfaces from nanoparticles.

The problem of the invention is solved by a composition comprising the ingredients as defined in claim 1. In particular, the problem of the invention is solved by a composition for decontamination of skin from nanoparticles, wherein said composition comprises
- 1 to 40 % by weight of at least one water-soluble polymer,
- 1 to 40 % by weight of at least one phyllosilicate,
- 1 to 30 % by weight charcoal and/or graphite, and
- water.

In a preferred embodiment, the water-soluble polymer is selected from the group consisting of polyethylene glycol, polypropylene glycol, polyvinyl pyrrolidone, carboxymethyl cellulose, polyvinyl alcohol, polyacrylic acid, chitosan, dextran, guar gum, hyaluronic acid, polyethylene imine, polyvinyl polypyrrolidon (PVPP), proteins such as bovine serum albumin (BSA), DMSO, polygelin, polyacryl amide, polyphosphate (sodium polyphosphate), polyphosphazene, xanthan, pectin, carrageenan and glycerin. The function of these water-soluble polymers is to create an environment that is more hydrophobic than pure water.

In a more preferred embodiment, the water-soluble polymer comprised in the composition of the invention is polyethylene glycol (PEG).

PEG is an oligomer or polymer composed of ethylene oxide monomers. Because different applications require different polymer chain lengths, PEGs are prepared by polymerization of ethylene oxide and are commercially available over a wide range of molecular weights from 300 g/mol to 10,000,000 g/mol. While PEGs with different molecular weights find use in different applications, and have different physical properties (e.g. viscosity) due to chain length effects, their chemical properties are nearly identical. Different forms of PEG are also available, depending on the initiator used for the polymerization process - the most common initiator is a monofunctional methyl ether PEG, or methoxypoly(ethylene glycol), abbreviated mPEG. Lower-molecular-weight PEGs are also available as purer oligomers, referred to as monodisperse, uniform, or discrete.

PEGs are also available with different geometries:
- Linear PEGs, where the ethylene oxide monomers are bound to each other in an unbranched polymer chain;
- Branched PEGs, which have three to ten PEG chains emanating from a central core group;
- Star PEGs, which have 10 to 100 PEG chains emanating from a central core group; and
- Comb PEGs, which have multiple PEG chains normally grafted onto a polymer backbone.

The numbers that are often included in the names of PEGs indicate their average molecular weights (e.g. a PEG with n = 9 would have an average molecular weight of approximately 400 daltons, and would be labeled PEG 400). Most PEGs include molecules with a distribution of molecular weights (i.e. they are polydisperse). The size distribution can be characterized statistically by its weight average molecular weight (Mw) and its number average molecular weight (Mn), the ratio of which is called the polydispersity index (Mw/Mn). MW and Mn can be measured by mass spectrometry.

PEG is soluble in water, methanol, ethanol, acetonitrile, benzene, and dichloromethane, and is insoluble in diethyl ether and hexane.

In one embodiment, the composition of the invention may comprise a starPEG. Suitably, said starPEG has a molecular weight in the range of 4 kD to 40 kD, preferably in the range of 4 kD to 30 kD, more preferably in the range of 4 kD to 20 kD, most preferably in the range of 4 kD to 10 kD. Suitably, said starPEG is a 4-arm starPEG.

In a more preferred embodiment, the composition of the invention comprises a linear PEG. Using linear PEGs has the advantage that linear PEGs are cheaper and possess a narrower molecular weight distribution. The use of linear PEG in the composition according to the invention has been found to be especially suitable for the decontamination of nanoparticles from skin.

Ingredients of the composition of the invention are preferred that do not penetrate into the skin and/or which do not facilitate the penetration of hazardous substances, such as nanoparticles into and through the skin. PEGs with higher molecular weights are therefore advantageous, because these PEGs show substantially no skin penetration. Therefore, in a most preferred embodiment, the PEG comprised in the composition according to the invention, in particular the linear PEG, has a molecular weight of more than 200 Da, preferably of 1 kDa or higher. Even most preferably, the linear PEG comprised in the composition according to the invention has a molecular weight selected from 5 kDa or higher, 6 kDa or higher, 7 kDa or higher, or 8 kDa or higher.

In a further preferred embodiment, the PEG, which is used to prepare the composition of the invention, may be functionalized. By "functionalize" is meant to modify a molecule in a manner that results in the attachment of a functional group or moiety. For example, a molecule may be functionalized by the introduction of a molecule which makes the molecule a strong nucleophile or a conjugated unsaturation. Preferably a molecule, for example PEG, is functionalized to become a thiol, amine, acrylate, azide, alkyne, quinone, maleimide, or carboxylic acid. This type of functionalization may be advantageous in the removal of nanoparticles from skin.

Good results in the removal of hazardous substances from skin have been achieved, when the composition of the invention comprises 1 to 40 % by weight of at least one water-soluble polymer, in particular a PEG as described herein. Preferably, the amount of the at least one water-soluble polymer, in particular a PEG as described herein in the composition of the invention is in the range between 10 % by weight and 40 % by weight or 15 % by weight and 40 % by weight, more preferably between 20 % by weight and 40 % by weight or 25 % by weight and 40 % by weight, most preferably between 30 % by weight and 40 % by weight or 35 % by weight and 40 % by weight. Best results in skin decontamination from nanoparticles has been achieved, when the amount of the at least one water-soluble polymer, in particular a PEG as described herein, in the composition of the invention is in the range between 30 % by weight and 35 % by weight, such as 31 % by weight, 32 % by weight, 33 % by weight and 34 % by weight. With such compositions it was possible to remove more than 85 %, and up to 100 % of the nanoparticles from a treated skin.

Therefore, in the invention provides in a preferred embodiment a for decontamination of skin from hazardous substances, wherein said composition comprises
- 30 to 35 % by weight of at least one water-soluble polymer, preferably a PEG,
- 1 to 40 % by weight of at least one phyllosilicate,
- 1 o 30 % by weight charcoal and/or graphite, and
- water.

Phyllosilicates are sheet silicate minerals, formed by parallel sheets of silicate tetrahedra with Si₂O₅ or a 2:5 ratio. Examples of phyllosilicates suitable for use in the composition of the invention are ajoite, allophane, annite, apophyllite, apophyllite-(kf), bentonite, biotite, bowenite, brammallite, carletonite, caryopilite, cavansite, chamosite, chapmanite, chrysocolla, clay, clay mineral, clintonite, cymrite, delessite, dickite, ekanite, ephesite, expanded clay aggregate, fraipontite, franklinphilite, fuchsite, greenalite, gyrolite, halloysite, hisingerite, imogolite, kampfite, kaolinite, kegelite, kerolite, lepidolite, macaulayite, macdonaldite, magadiite, medicinal clay, meerschaum pipe, metal clay, mica, minnesotaite, nelenite, neptunite, okenite, organoclay, pentagonite, petalite, phlogopite, pimelite, pyrophyllite, sanbornite, searlesite, sepiolite, seraphinite, sericite, sericitic alteration, siderophyllite, soapstone, stilpnomelane, talc, thuringite, tumchaite, tuperssuatsiaite, ussingite, zakharovite, and zussmanite. The phyllosilicates immobilize the nanoparticles and can thus be washed off to facilitate the decontamination of the skin.

Suitably, the at least one phyllosilicate comprised in the composition of the invention is selected from the group consisting of kaolin, bentonite, vermiculite, talc, mica, sepiolite, palygorskite, saponite, hectorite, smectite, montmorillonite K-10, aquamont-CA, illite, montmorillonite KSF and aquamont B, or a mixture of two or more thereof.

In a most preferred embodiment, the at least one phyllosilicate comprised in the composition of the invention is bentonite or kaolin or a mixture thereof, suitably bentonite.

Good results in the removal of hazardous compounds such as nanoparticles from skin have been achieved, when the composition of the invention comprises 2 to 30 % by weight of at least one phyllosilicate, in particular bentonite, as described herein. Preferably, the amount of the at least one phyllosilicate or a mixture of phyllosilicates, in particular bentonite or mixtures including bentonite, as described herein, in the composition of the invention is 10 wt. % or higher, e.g. in the range between 10 % by weight and 30 % by weight or 12 % by weight and 30 % by weight, more preferably between 14 % by weight and 30 % by weight or 16 % by weight and 30 % by weight, most preferably between 16 % by weight and 25 % by weight or 16 % by weight and 20 % by weight. Best results in skin decontamination from nanoparticles has been achieved, when the amount of the at least one phyllosilicate or a mixture of phyllosilicates, in particular bentonite or a mixture including bentonite, as described herein, in the composition of the invention is 17 % by weight 18 % by weight or 19 % by weight. With such compositions it was possible to remove more than 85 %, and up to 100 % of the nanoparticles from a treated skin.

Therefore, in the invention provides in a preferred embodiment a for decontamination of skin from hazardous compounds, wherein said composition comprises
- 1 to 40 % by weight of at least one water-soluble polymer,
- 16 to 25 % by weight of at least one phyllosilicate, preferably bentonite and/or kaolin;
- 1 o 30 % by weight charcoal and/or graphite, and
- water.

The composition of the invention further comprises charcoal and/or graphite. Any type of charcoal may be suitable for use in the composition of the invention. The charcoal is preferably an activated carbon. The charcoal, such as activated carbon, has a large surface area to bind non-specific substances and depending on the grain size, some additional friction can be generated, resulting in a light peeling effect.

When the composition of the invention comprises activated carbon, any type of activated carbon may be used. Preferred activated carbon may be made from hard coal, coconut or peat.

The average particle size of the charcoal such as activated carbon used in the composition of the invention is preferably in the range between 3 and 50 µm, more preferably 4 and 40 µm, most preferably between 5 and 30 µm. Best results in skin decontamination from hazardous substances has been achieved, when the average particle size of the charcoal such as activated carbon used in the composition of the invention is 6 µm, 10 µm, 15 µm, 20 µm or 25 µm. With such compositions it was possible to remove more than 85 %, and up to 100 % of e.g. nanoparticles from a treated skin.

When the composition of the invention comprises graphite, any type of graphite may be used. A preferred graphite has a particle size d₅₀ of 80 µm or lower, preferably of 60 µm or lower, more preferably of 40 µm or lower, most preferably of 20 µm or lower. Suitable commercially available products are Graphite Aldrich <20µm and Graphite Alpha Aesar 200 mesh.

The composition of the invention may comprise either activated carbon or graphite or mixtures thereof.

Good results in the removal of hazardous compounds, such as nanoparticles, from skin have been achieved, when the composition of the invention comprises 1 to 30 % by weight of a activated carbon, graphite or a mixture thereof, as described herein. Preferably, the amount of activated carbon, graphite or a mixture thereof, as described herein, in the composition of the invention is 10 wt. % or higher, e.g. in the range between 12 % by weight and 30 % by weight or 14 % by weight and 30 % by weight, more preferably between 16 % by weight and 30 % by weight or 18 % by weight and 30 % by weight, most preferably between 20 % by weight and 30 % by weight. Best results in skin decontamination from hazardous compounds, such as nanoparticles, has been achieved, when the amount of the activated carbon, graphite or a mixture thereof, as described herein, in the composition of the invention is 9 % by weight or higher, 10 % by weight, 11 % by weight, 12 % by weight, 13 % by weight, 14 % by weight or 15 % by weight. With such compositions it was possible to remove more than 85 %, and up to 100 % of the nanoparticles from a treated skin.

Accordingly, the composition of the invention comprises in a preferred embodiment
- 1 to 40 % by weight of at least one water-soluble polymer,
- 2 to 30 % by weight of at least one phyllosilicate,
- ≥10 % by weight charcoal and/or graphite, and
- water.

In a more preferred embodiment, the invention provides a composition for decontamination of skin from nanoparticles, wherein said composition comprises
- 30 to 35 % by weight of at least one water-soluble polymer;
- 16 to 25 % by weight of at least one phyllosilicate;
- ≥10 % by weight charcoal and/or graphite, and
- water.

In a most preferred embodiment, the invention provides a composition for decontamination of skin from nanoparticles, wherein said composition comprises
- 30 to 35 % by weight of a PEG;
- 16 to 25 % by weight of bentonite and/or kaolin;
- ≥10 % by weight activated carbon and/or graphite, and
- water.

In certain embodiments, compositions of the present invention are sterile and preservative-free. In other embodiments, compositions of the present invention optionally comprise a preservative. In particular embodiments, a preservative is a paraben-free preservative. Parabens are a series of parahydroxybenzoates or esters of parahydroxybenzoic acid and are known to cause cytokine release and irritation and have been linked to several types of cancer. Examples of parabens include methylparaben, ethylparaben, propylparaben, butylparaben, heptylparaben, isobutylparaben, isopropylparaben, benzylparaben, and their sodium salts.

Exemplary paraben-free preservatives suitable for use in the present invention include phenethyl alcohol, caprylyl glycol, phenoxyethanol, a sorbate, potassium sorbate, sodium sorbate, sorbic acid, sodium benzoate, benzoic acid, acemannan, oleuropein, carvacrol, cranberry extract, gluconolactone, green tea extract, *Helianthus annuus* seed oil, *Lactobacillus* ferment, *Usnea barbata* extract, polyaminopropyl biguanide, polyglyceryl-3 palmitate, polyglyceryl-6 caprylate, pomegranate extract, *Populus tremuloides* bark extract, resveratrol, *Rosmarinus oificinalis* leaf extract, benzyl alcohol, or any combination thereof.

The composition of the invention may further comprise a gelation or viscosity increasing agent, which is preferably non-irritating. Examples of non-irritating gelation agents include a cellulose ether (e.g., methyl cellulose, carboxymethyl cellulose, hypromellose, ethylcellulose, ethyl methyl cellulose, hydroxyethyl cellulose, hydroxypropyl cellulose, hydroxyethyl methyl cellulose, ethyl hydroxyethyl cellulose, or a combination thereof), xanthan carrageenan, carbomer, polyoxazoline, or any combination thereof. Preferred gelation agents are carboxymethyl cellulose and xanthan carrageenan.

The amount of the preservatives in the composition of the invention is preferably up to 1 % by weight, more preferably 0.1, 0.2, 0.3, 0.4, 0.5, 0.6, 0.7, 0.8, 0.9 or 1.0 % per weight.

The amount of the gelation agent in the composition of the invention is preferably in the range of 5 to 10 % by weight, more preferably 5 %, 6 %, 7 %, 8 %, 9 % or 10 % by weight.

In a further embodiment, the composition of the invention further comprises
- up to 1 % by weight preservatives; and/or
- 1 to 10 % by weight of a gelation agent.

Accordingly, the composition of the invention comprises in one embodiment
- 1 to 40 % by weight of at least one water-soluble polymer,
- 1 to 40 % by weight of at least one phyllosilicate,
- 1 to 30 % by weight charcoal and/or graphite,
- up to 1 % by weight preservatives; and/or
- 1 to 10 % by weight of a gelation agent; and
- water.

In a more preferred embodiment, the invention provides a composition for decontamination of skin from nanoparticles, wherein said composition comprises
- 30 to 35 % by weight of at least one water-soluble polymer;
- 16 to 25 % by weight of at least one phyllosilicate;
- ≥10 % by weight charcoal and/or graphite;
- up to 1 % by weight preservatives; and/or
- 1 to 10 % by weight of a gelation agent; and
- water.

In a most preferred embodiment, the invention provides a composition for decontamination of skin from nanoparticles, wherein said composition comprises
- 30 to 35 % by weight of a PEG;
- 16 to 25 % by weight of bentonite and/or kaolin;
- ≥10 % by weight activated carbon and/or graphite;
- up to 1 % by weight preservatives; and/or
- 1 to 10 % by weight of carboxymethyl cellulose and/or xanthan carrageenan; and
- water.

Water is primarily used as a solvent in the composition of the invention, in which it dissolves many of the ingredients described herein. The water also forms emulsions in which lipophilic and hydrophilic components of the composition are combined to form creams and lotions. The water used in the composition of the invention is selected from tap water, purified water, deionized water and distilled water. Preferably the water has been sterilized before its use to manufacture the composition of the invention. The final amount of water comprised in the composition of the invention is calculated based on the sum of the amounts of the other ingredients. The sum amounts of the other ingredients are calculated in % by weight and the difference to 100 % per weight of the composition of the invention is compensated with water.

A typical amount of water comprised in the composition of the invention is in the range between 30 and 60 % by weight, preferably between 35 and 55 % by weight, more preferably between 40 to 50 % by weight, most preferably between 40 and 45 % by weight, such as 41, 42, 43 or 44 % by weight.

In a further preferred embodiment, the composition of the invention does not contain surfactants. Therefore, the composition of the invention is particularly advantageous, because the composition itself does not penetrate into and/or through the skin. A further advantage of the composition of the invention is that it does not affect the barrier function of the skin. In particular, the composition of the invention does not widen the pores of the skin, as this favors the diffusion of hazardous particles through the skin. Widening of skin pores has for example recognized with conventional soap. Accordingly, the composition of the invention does also not facilitate the penetration of hazardous substances, such as nanoparticles, microplastic particles or viruses, into and through the skin. Also, after treating a skin area which is contaminated with hazardous substances , with the composition of the invention, there is no need for a subsequent skin care, because the skin is not damaged by the composition of the invention in any way.

As discussed herein, the composition of the invention is particularly suitable for the decontamination of skin from nanoparticles. In its 2012 proposed terminology for biologically related polymers, the IUPAC defined a nanoparticle as "a particle of any shape with dimensions in the 1 x 10⁻⁹ and 1 x 10⁻⁷ m range" (Vert, M.; Doi, Y.; Hellwich, K. H.; Hess, M.; Hodge, P.; Kubisa, P.; Rinaudo, M.; Schué, F. O. (2012). "Terminology for biorelated polymers and applications (IUPAC Recommendations 2012)". Pure and Applied Chemistry. 84 (2): 377-410). In another 2012 publication, the IUPAC extends the term to include tubes and fibers with only two dimensions below 100 nm.

In common usage, "nanoscale" is usually understood to be the range from 1 to 100 nm because the novel properties that differentiate particles from the bulk material typically develop at that range of sizes.

Nanoparticles can e.g. be metallic nanoparticles, inorganic nanoparticles, organic nanoparticles and semi-conductive nanoparticles.

The composition of the invention has been shown to be effective in the decontamination of nanoparticles with the size range between 1 to 100 nm, and to be particularly effective in the decontamination of very small nanoparticles, such as quantum dots, with a size in the range of 4 to 8 nm.

The composition of the invention is suitably provided as a formulation that allows an easy administration of the composition on contaminated skin areas. Typical formulations, which fulfill this purpose, are creames, gels or lotions.

A lotion is a low-viscosity topical preparation intended for application to the skin. By contrast, creams and gels have higher viscosity, typically due to lower water content. Lotions are applied to external skin with bare hands, a brush, a clean cloth, or cotton wool.

A cream is a preparation usually for application to the skin. Creams may be considered pharmaceutical products or even cosmetic creams. Creams are semi-solid emulsions of oil and water. They are divided into two types: oil-in-water (O/W) creams which are composed of small droplets of oil dispersed in a continuous water phase, and water-in-oil (W/O) creams which are composed of small droplets of water dispersed in a continuous oily phase. Oil-in-water creams are more comfortable and cosmetically acceptable as they are less greasy and more easily washed off using water.

A gel is a solid that can have properties ranging from soft and weak to hard and tough. Gels are defined as a substantially dilute cross-linked system, which exhibits no flow when in the steady-state. By weight, gels are mostly liquid, yet they behave like solids due to a three-dimensional cross-linked network within the liquid. It is the crosslinking within the fluid that gives a gel its structure (hardness) and contributes to the adhesive stick (tack). In this way, gels are a dispersion of molecules of a liquid within a solid medium.

In a preferred embodiment, the composition of the invention is formulated as a cream.

In another preferred embodiment, the composition of the invention is formulated as a lotion.

In a further preferred embodiment, the composition of the invention is formulated as a gel.

In a further aspect, the invention provides an application device, which comprises a composition of the invention. Depending on the type of the formulation (cream, gel, lotion) a suitable application device is selected e.g. from a sponge, cloth, shoe cleaning tube, spray can or spray device. With the appropriate application device, it is possible to administer the respective formulation very precisely locally. Suitable application devices are known to person skilled in the art from the fields of pharmaceuticals, cosmetic and cleaning supplies.

Further advantageous in regard to the composition and application device of the invention is that the composition itself functions as a cleaning indicator. Said indicator shows whether the affected area of the skin has been cleaned sufficiently and effectively. In a more preferred embodiment, the activated carbon and/or graphite comprised in the composition of the invention act as cleaning indicator. After administration of the composition of the invention on contaminated skin and the expiry of a predetermined exposure time, the composition of the invention is washed off from the skin with water. Cleaning of the skin from the composition of the invention, accompanied by the cleaning of the skin from the nanoparticle contamination, is easily visible and indicated by the washing off of any residual activated carbon and/or graphite components from the skin. If the composition is not completely washed off, black spots can be seen in the skin pores.

Accordingly, the invention provides in a further embodiment an application device, which comprises a composition of the invention, and wherein said application device or composition optionally further comprises an indicator, wherein said indicator shows whether the affected area of the skin has been cleaned sufficiently and effectively.

In a more preferred embodiment said application device comprising a cleaning indicator is a sponge. More preferably, said composition comprised in the sponge is formulated as a gel, wherein said gel, in particular the activated carbon and/or graphite comprised in said gel, indicates whether the affected area of the skin has been cleaned sufficiently and effectively.

The cream, lotion or gel can be applied to contaminated skin and rubbed into the skin, suitably with a sponge or a tube after contamination of the skin with nanoparticles. The cream, lotion or gel binds the nanoparticles and can then be washed off easily with water together with the particles.

The composition of the invention does not comprise any pharmaceutically active ingredients. The composition of the invention is therefore considered as a non-medical composition or preparation. In a further preferred embodiment, the composition is a cosmetic composition or preparation.

The invention further provides a kit. Said kit comprises the application device of the invention and instructions for use of the kit and the application device. The application device in the kit is preferably a sponge, which comprises the decontamination composition of the invention. The decontamination composition comprised by the sponge of the kit is preferably in the form of a gel.

In a further aspect, the invention relates to the use of the composition or the application device of the invention in the decontamination of skin from hazardous substances, such as nanoparticles, microplastic particles or viruses, preferably nanoparticles.

In a preferred embodiment, the invention relates to the non-medical use of the composition or the application device of the invention in the decontamination of skin from hazardous substances, such as nanoparticles, microplastic particles or viruses, preferably nanoparticles. Said non-medical use is preferably the use for cleaning purposes, i.e. the cleaning of skin from contaminations with hazardous substances, such as nanoparticles, microplastic particles or viruses, preferably nanoparticles.

In a further preferred embodiment, the invention provides said composition or application device for use in the decontamination of skin from hazardous substances, such as nanoparticles, microplastic particles or viruses, preferably nanoparticles.

In a further preferred embodiment, the invention relates to the use of said composition or application device for the preparation of a medicament for treatment of contaminated skin, in particular for the decontamination of skin from hazardous substances, such as nanoparticles, microplastic particles or viruses, preferably nanoparticles.

In a further preferred embodiment, the invention relates to a method of treating skin that is contaminated with hazardous substances, such as nanoparticles, microplastic particles or viruses, preferably nanoparticles, said method comprising the steps of administering the composition of the invention on said contaminated skin of a subject in need thereof and decontamination of the skin from the hazardous substances, such as nanoparticles, microplastic particles or viruses, preferably nanoparticles.

The use or method for decontamination of the skin from hazardous substances, such as nanoparticles, microplastic particles or viruses, preferably nanoparticles generally comprises the steps of:
- applying the decontamination composition of the invention on the contaminated area of the skin, preferably by rubbing with suitable means, such as a sponge;
- washing off the decontamination composition together with the hazardous substances, such as nanoparticles, microplastic particles or viruses, preferably nanoparticles with water, preferably cold water;
- optionally performing a quality control to ensure that the hazardous substances, such as nanoparticles, microplastic particles or viruses, preferably nanoparticles were washed off completely by investigating with the naked eye, whether the charcoal or graphite contained in the decontamination composition was washed off from the skin completely.

The invention is further illustrated by 6 figures and 8 examples.

### Brief description of the Figures

- **Figure 1**: shows a graphic representation of the content of activated carbon, bentonite and kaolin vs. their respective decontamination efficiencies of nanoparticles from skin.
- **Figure 2**: shows the results of the comparison of decontamination efficiency of the composition of the invention with commercial cleansing products.
- **Figure 3**: shows at the left hand side the decontamination composition directly after application, in the middle the decontamination composition after insufficient washing off, residues of the black activated carbon particles are still visible; and at the right hand side the completely decontaminated skin after sufficient washing.
- **Figure 4**: shows an example of a decontamination kit of the invention.
- **Figure 5**: shows the results of time-dependent decontamination efficacy tests.
- **Figure 6**: shows the percentage of nanoparticles remaining on the skin after decontamination.

### Examples of the invention

### Example 1: General manufacturing process of the decontamination composition

In a first step, at least one water-soluble polymer and optionally a gelation agent are mixed with purified water. Then, the at least one activated phyllosilicate and the activated carbon are homogenized and after homogenization added to the wet mixture of the at least one water-soluble polymer and optionally the gelation agent in water. The so completed mixture is thoroughly stirred until homogenization.

Further described is the representative manufacture of 100 g of a gel:
24 g of PEG and 6 g carboxymethyl cellulose is mixed with 31 g of purified water (18.2 MΩ). Then, 13 g of Bentonite, 13 g of Kaolin, and 13 g of activated carbon are homogenized before adding to the wet mixture. The completed mixture is thoroughly stirred until homogenization.

### Example 2: General design of the decontamination experiments

Decontamination experiments were performed with porcine skin.

### Preparation of porcine skin

Porcine skin was prepared as follows:
Pigs of the German Landrace are used, aged between 5 and 8 months. The age, sex and weight of each donor animal were documented in the test protocol.

Pieces of skin of approximately 10 cm x 10 cm were removed from the area of the lateral abdominal wall with the help of a scalpel. A distance of at least 5 cm from the spinal column, ribs and the middle of the abdomen were maintained. The pieces of skin are wrapped in surgical drapes to avoid contamination with subcutaneous fatty tissue. The transport to the laboratory is carried out in freezer bags in a cool box at approx. 4-8 °C on cooling batteries or ice.

First, the skin surface is cleaned with lukewarm water. Then the preparation base is covered with a layer of aluminium foil and above that a layer of absorbent material (e.g. Zemuko universal compress). A piece of skin is placed on top of this with the stratum corneum side down. Artery clamps are attached to the four corners of the skin, and the skin is stretched by means of the rubber bands. Alternatively, the skin can be fixed with four needles which are inserted through the skin corners into a polystyrene pad. The subcutaneous fatty tissue is grasped with surgical tweezers and separated directly below the dermis with a scalpel. Care must be taken that no contact of the surface with subcutaneous fat occurs. Due to the risk of contamination, the outermost 5 mm in the marginal area must be discarded after the subcutaneous fat tissue has been dissected. The prepared skin is then placed on the aluminium foil with anatomical tweezers, smoothed if necessary, and wrapped in aluminium foil and placed in a freezer bag. The freezer bag is sealed as airtight as possible. The skin is frozen at - 20°C to -30°C in the freezer on a flat surface. For further use, the samples shall be stored in the freezer for at least 24 hours. The maximum storage period is 6 months. A documentation of the storage period must be provided.

Before use in the experiments, the skin pieces must be hydrated. For hydration, a beaker with acceptor medium is tempered to 32 °C. The skin pieces are then placed in the beaker and completely covered by the acceptor medium.

### Decontamination experiments

Skin samples were contaminated with 2 to 6 nm CdSe quantum dot particles, which are particularly difficult to remove due to their small size and are therefore representative for a good decontamination efficacy also for nanoparticles of larger size. Due to their fluorescence the quantum dot particles are quantitatively well detectable. After an exposure time of 30 seconds after contamination appropriate countermeasures were taken. These werethorough rubbing with the composition of the invention and subsequent washing with cold water. All pieces of skin were examined in a fluorescence spectrometer.

### Example 3: Screening of effective amounts of the composition ingredients

Porcine skin pieces were contaminated with nanoparticles and treated different decontamination compositions as described in example 2. The results are shown in table 1.

**Table 1: Efficiency of different decontamination compositions**

| **Decontamination Efficiency, %** | **Ingredient 1** | **wt%** | **Ingredient 2** | **wt%** |
|---|---|---|---|---|
| 69,0 | PEG | 100 | None | |
| 75,4 | PEG | 43 | Water | 57 |
| 37,8 | Polypropylene glycol | 100 | None | |
| 96,8 | Water | 65 | Activated carbon | 35 |
| 78,9 | Water | 60 | Pumice stone flour | 40 |
| 81,5 | Water | 83 | Kaolin | 17 |
| 27,1 | Water | 78 | Bentonite | 22 |
| 33,2 | Water | 90 | Hyaluronic acid | 10 |
| 36,6 | Water | 50 | Polyethylene imine | 50 |
| 61,7 | Water | 94 | Polyvinyl alcohol | 6 |
| 54,2 | Water | 50 | Polyvinyl pyrrolidone | 50 |
| 82,5 | Water | 50 | Polyacrylic acid | 50 |
| 4,3 | PEG | 99 | Activated carbon | 1 |
| 14,2 | PEG | 95 | Activated carbon | 5 |
| 68,6 | PEG | 90 | Activated carbon | 10 |
| 97,3 | PEG | 80 | Activated carbon | 20 |
| 97,7 | PEG | 70 | Activated carbon | 30 |
| 72,5 | PEG | 99 | Bentonite | 1 |
| 81,7 | PEG | 90 | Bentonite | 10 |
| 89,0 | PEG | 80 | Bentonite | 20 |
| 90,6 | PEG | 70 | Bentonite | 30 |
| 78,7 | PEG | 60 | Bentonite | 40 |
| blank | PEG | 50 | Bentonite | 50 |
| 80,3 | PEG | 99 | Kaolin | 1 |
| 79,1 | PEG | 90 | Kaolin | 10 |
| 84,5 | PEG | 80 | Kaolin | 20 |
| 60,2 | PEG | 70 | Kaolin | 30 |
| 40.1 | Water | 70 | Talc | 30 |
| 47.1 | Water | 70 | Montmorillonite | 30 |
| 41.5 | Water | 70 | Illite | 30 |

The results in table 1 show that mixtures of PEG, a water-soluble polymer, with phyllosilicates, such as bentonite or kaolin, and activated carbon are generally more efficient in the decontamination of nanoparticles from skin.

### Example 4: Optimization of the decontamination composition

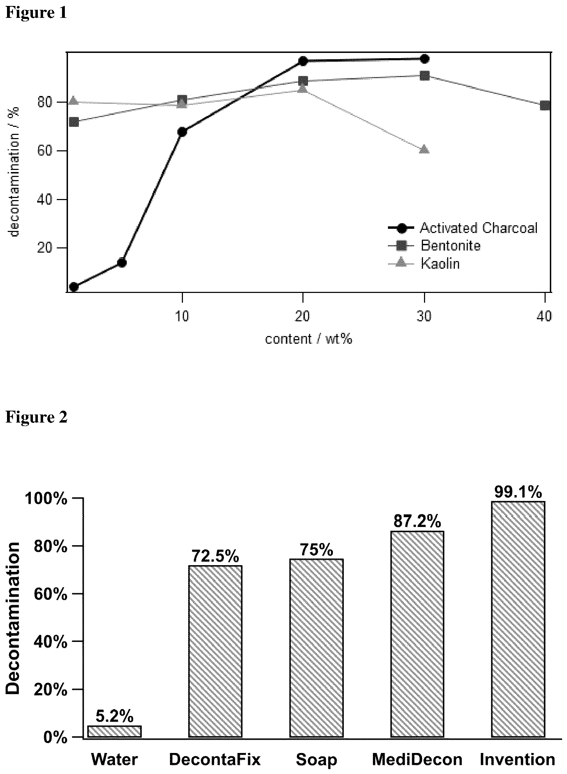

Based on the results achieved in example 3, the decontamination compositions were further optimized in regard to the specific ingredients and their amounts contained in the compositions. Efficacy tests were performed in triplicates as described in example 2. Compositions were prepared as described in example 1. The results are shown in table 2. Figure 1 shows a graphic representation of the content of activated carbon, bentonite and kaolin vs. their respective decontamination efficiencies of nanoparticles from skin.

**Table 2: Efficiency of optimized decontamination compositions**

| **Decontamination Efficiency, %** | **Ingredient 1** | **wt%** | **Ingredient 2** | **wt%** | **Ingredient 3** | **wt%** | **Ingredient 4** | **wt%** | **Ingredient 5** | **wt%** |
|---|---|---|---|---|---|---|---|---|---|---|
| 95.5 | PEG | 31 | Water | 42 | Activated carbon | 9 | Kaolin | 9 | Bentonite | 9 |
| 98.5 | PEG | 31 | Water | 42 | Activated carbon | 9 | Kaolin | 9 | Bentonite | 9 |
| 97.2 | PEG | 31 | Water | 42 | Activated carbon | 9 | Kaolin | 9 | Bentonite | 9 |
| 97.9 | PEG | 31 | Water | 42 | Graphite ¹ | 9 | Kaolin | 9 | Bentonite | 9 |
| 96.5 | PEG | 31 | Water | 42 | Graphite ¹ | 9 | Kaolin | 9 | Bentonite | 9 |
| 98.1 | PEG | 31 | Water | 42 | Graphite ¹ | 9 | Kaolin | 9 | Bentonite | 9 |
| 98.3 | PEG | 31 | Water | 42 | Graphite ² | 9 | Kaolin | 9 | Bentonite | 9 |
| 92.3 | PEG | 31 | Water | 42 | Graphite ² | 9 | Kaolin | 9 | Bentonite | 9 |
| 88.4 | PEG | 31 | Water | 42 | Graphite ² | 9 | Kaolin | 9 | Bentonite | 9 |
| 100 | PEG | 31 | Water | 42 | Activated carbon ³ | 9 | Kaolin | 9 | Bentonite | 9 |
| 98.8 | PEG | 31 | Water | 42 | Activated carbon ³ | 9 | Kaolin | 9 | Bentonite | 9 |
| 100 | PEG | 31 | Water | 42 | Activated carbon ³ | 9 | Kaolin | 9 | Bentonite | 9 |
| 99.9 | PEG | 31 | Water | 42 | Activated carbon ⁴ | 9 | Kaolin | 9 | Bentonite | 9 |
| 100 | PEG | 31 | Water | 42 | Activated carbon ⁴ | 9 | Kaolin | 9 | Bentonite | 9 |
| 100 | PEG | 31 | Water | 42 | Activated carbon ⁴ | 9 | Kaolin | 9 | Bentonite | 9 |
| 98.5 | PEG | 31 | Water | 42 | Activated carbon ³ | 9 | Kaolin | 9 | Bentonite | 9 |
| 99.7 | PEG | 31 | Water | 42 | Activated carbon ³ | 9 | Kaolin | 9 | Bentonite | 9 |
| 98.9 | PEG | 31 | Water | 42 | Activated carbon ³ | 9 | Kaolin | 9 | Bentonite | 9 |
| 98.3 | PEG | 31 | Water | 42 | Activated carbon ³ | 9 | Kaolin | 9 | Bentonite | 9 |
| 96.6 | PEG | 31 | Water | 42 | Activated carbon ³ | 9 | Kaolin | 9 | Bentonite | 9 |
| 95.7 | PEG | 31 | Water | 42 | Activated carbon ³ | 9 | Kaolin | 9 | Bentonite | 9 |
| 99.1 | PEG | 44.6 | Water | 17.8 | Activated carbon | 12.5 | Kaolin | 12.5 | Bentonite | 12.5 |
| 87.3 | Dextran | 31 | Water | 42 | Activated carbon | 9 | Kaolin | 9 | Bentonite | 9 |

| | | | | | | | | | | |
|---|---|---|---|---|---|---|---|---|---|---|
| ¹ Graphite Aldrich < 20 µm, ² Graphite Alfa Aesar 200 mesh, ³ Activated carbon Norit SX Super E153 8033.8; ⁴ Norit SX Super E153 8017.7 | | | | | | | | | | |

The results in table 2 show that compositions comprising 31 % per weight PEG, 42 % per weight water, 9 % per weight activated carbon or graphite, 9 % per weight kaolin and 9 % per weight bentonite are highly effective showing decontamination rates of 88.4 % up to 100 %.

### Example 5: Decontamination of skin from microplastic particles

The invention was also tested for the decontamination of microplastic particles. For this, polystyrene microplastic particles with a diameter of 3 µm (purchased from polysciences.com) were labelled with nile red to enable the detection via fluorescence. (Erni-Cassola, G. et al., Lost, but found with Nile red; a novel method to detect and quantify small microplastics (20 µm-1 mm) in environmental samples. Environ. Sci. Technol., Publication Date (Web): 07 Nov 2017, 27 pages, doi:10.1021/acs.est.7b04512) The decontamination experiments were conducted as described in example 2. The decontamination efficiency by the invention was 100%.

### Example 6: Comparison of decontamination efficiency with commercial products

Porcine skin was prepared as described in example 2. A composition of the invention comprising 31 % per weight PEG, 42 % per weight water, 9 % per weight activated carbon or graphite, 9 % per weight kaolin and 9 % per weight bentonite was prepared as described in example 1. Decontamination experiments were performed as described in example 2.

The measurements show that washing with water removes only 5.2% of the particles from the skin, treatment with water and soap removes 75% of the particles. The composition of the invention was able to remove 99.1% of the particles from the skin, and thus, was more efficient than soap and water, but was also more efficient than other special products used for decontamination of the skin (Decontafix and MediDecon), which were able to remove 72.5% and 87.2% of the particles from the skin. These results are shown in figure 2.

Conventional cleansing products such as soap and water, cause the skin pores to dilate and are thus unsuitable. After a large series of tests with various water-soluble plastics, polyethylene glycol showed the best decontamination ability. Since the decontamination capability alone was not sufficient, other ingredients were tested that further improved the decontamination performance. An important criterion was the compatibility of the components with each other in order to produce the composition of the invention in a functional way. Kaolin and bentonite (phyllosilicates or clay minerals), which primarily bind and immobilize charged nanoparticles in order to prevent them from skin penetration, were well suited for this purpose. In addition, the composition contains activated carbon, which has a very large surface area for non-specific binding of nanoparticles.

In addition, time-dependent decontamination tests after 30 s, 1 min, 2 min and 10 min were performed with the decontamination composition of the invention vs. a detergent (dish soap).

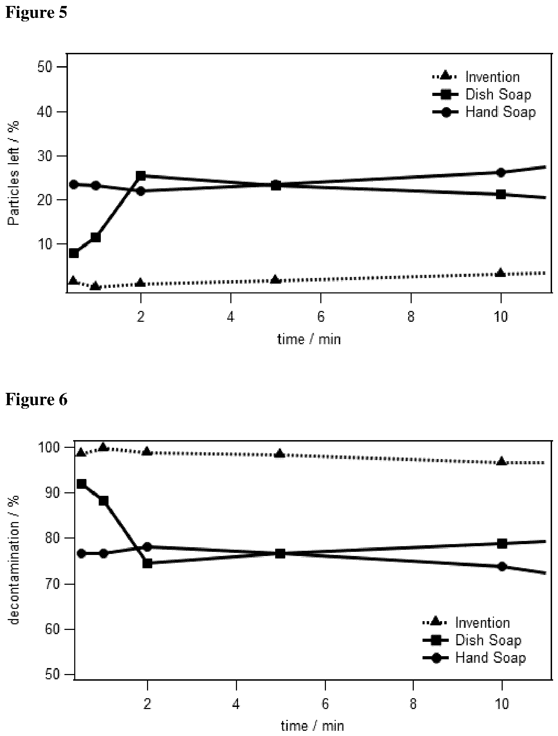

The results show that the detergent is less suitable for decontamination and decreases sharply in efficiency from 2 minutes, while the composition of the invention constantly remains high decontamination efficiency (figure 5). This coincides with percentage of the particles remaining on the skin (figure 6).

### Example 7: Indicator function of the decontamination composition

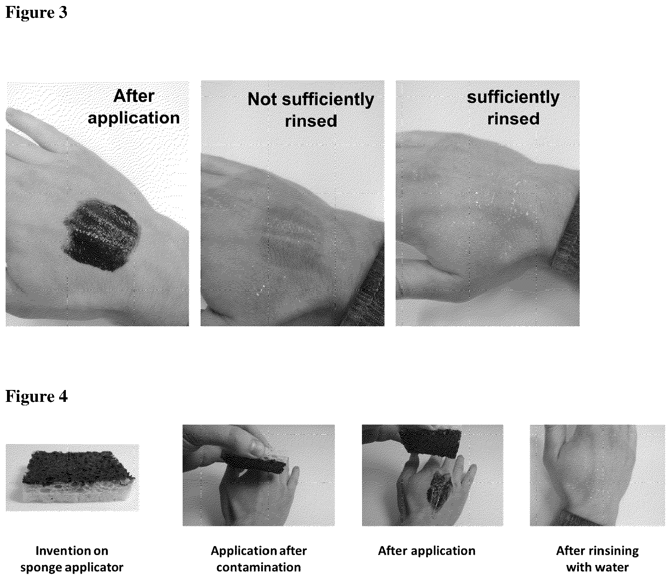

Activated carbon or graphite gives the composition a black color, which is an indicator of whether there is still composition with removed nanoparticles on the skin (see Figure 3).

After the skin has come into contact with nanoparticles, a sponge comprising the decontamination composition of the invention was used and the contaminated area of the skin was rubbed therewith, as shown in figure 3. Thereafter the decontamination composition together with the nanoparticles is washed off with cold water. In order to ensure that the rinsing is sufficiently long, the composition contains an indicator which allows with the naked eye to see when decontamination is complete. The indicator consists of activated carbon or graphite, which adheres to the pores of the skin. Since the activated carbon or graphite binds the nanoparticles, it can be seen that the decontamination is complete as soon as no activated carbon is visible.

### Example 8: Decontamination kit

An example of a decontamination kit of the invention comprises a sponge-like applicator impregnated with a decontamination composition of the invention in the form of a gel (see Figure 4). On the left hand side of figure 4, a sponge-like applicator is shown comprising the decomposition gel of the invention. The picture next to the left hand side picture shows the process of the application of the decomposition gel on a contaminated skin area. The third picture (seen from the left hand side) shows the skin after application of the decomposition gel. The picture at the right hand side shows the skin after complete washing off of the decomposition gel.

## Claims

1. A composition for decontamination of skin from hazardous substances, such as nanoparticles, microplastic particles and viruses, wherein said composition comprises
• 1 to 40 wt% of at least one water-soluble polymer,
• 1 to 40 wt% of at least one phyllosilicate,
• 1 to 30 wt% charcoal and/or graphite, and
• water.

2. The composition of claim 1, wherein said at least one water-soluble polymer is selected from polyethylene glycol, polypropylene glycol, polyvinyl pyrrolidone, carboxymethyl cellulose, polyvinyl alcohol, polyacrylic acid, chitosan, dextran, guar gum, hyaluronic acid, polyethylene imine, polyvinyl polypyrrolidon (PVPP), bovine serum albumin (BSA), DMSO, polygelin, polyacryl amide, polyphosphate (sodium polyphosphate), polyphosphazene, xanthan, pectin, carrageenan and glycerin.

3. The composition of claim 1 or 2, wherein said at least one polymer is polyethylene glycol or polypropylene glycol, preferably polyethylene glycol.

4. The composition of any one of claims 1 to 3, wherein said at least one phyllosilicate is selected from ajoite, allophane, annite, apophyllite, apophyllite-(kf), bentonite, biotite, bowenite, brammallite, carletonite, caryopilite, cavansite, chamosite, chapmanite, chrysocolla, clay, clay mineral, clintonite, cymrite, delessite, dickite, ekanite, ephesite, expanded clay aggregate, fraipontite, franklinphilite, fuchsite, greenalite, gyrolite, halloysite, hisingerite, imogolite, kampfite, kaolinite, kegelite, kerolite, lepidolite, macaulayite, macdonaldite, magadiite, medicinal clay, meerschaum pipe, metal clay, mica, minnesotaite, nelenite, neptunite, okenite, organoclay, pentagonite, petalite, phlogopite, pimelite, pyrophyllite, sanbornite, searlesite, sepiolite, seraphinite, sericite, sericitic alteration, siderophyllite, soapstone, stilpnomelane, talc, thuringite, tumchaite, tuperssuatsiaite, ussingite, zakharovite, and zussmanite.

5. The composition according to any of the preceding claims, wherein said at least one phyllosilicate is bentonite or kaolin, preferably bentonite.

6. The composition according to any of the preceding claims, wherein said charcoal is activated carbon, wherein said activated carbon is preferably made from hard coal, bituminated coal, coconut or peat.

7. The composition according to any of the preceding claims, comprising
• 1 to 40 wt% of at least one water-soluble polymer,
• 2 to 30 wt% of at least one phyllosilicate,
• ≥ 10 wt% charcoal and/or graphite, and
• water.

8. The composition according to any of the preceding claims, further comprising
• up to 1 wt% preservatives; and/or
• 1 to 10 wt% of a gelation agent.

9. The composition for use according to any of the preceding claims, wherein said composition does not contain surfactants and/or wherein said composition does not affect the barrier function of the skin.

10. The composition according to any one of the preceding claims, wherein said nanoparticles are selected from metallic nanoparticles, inorganic nanoparticles, organic nanoparticles and semiconductive nanoparticles.

11. The composition according to any of the preceding claims, wherein said composition is a cream, lotion or a gel.

12. The composition according to any of the preceding claims for use in the decontamination of skin from hazardous substances, such as nanoparticles, microplastic particles and viruses.

13. Application device comprising a composition according to any one of claims 1 to 11.

14. The application device according to claim 13, wherein said application device is selected from a sponge, cloth, shoe cleaning tube, spray can or spray device.

15. The application device according to claim 13 or 14, wherein said application device is a sponge, which comprises a composition according to any one of claims 1 to 11, wherein said composition is in the form of a gel, and wherein said application device or composition optionally further comprises an indicator, wherein said indicator shows whether the affected area of the skin has been cleaned sufficiently and effectively.
